# EUROPEAN PATENT APPLICATION

(11) **EP 4 494 689 A1**
(43) Date of publication of application: **22.01.2025**
(21) Application number: 24188130.9
(22) Date of filing: 11.07.2024
(51) Int. Cl.: A61M 39/10, A61M 39/26

(54) **CONNECTOR STRUCTURE**

(30) Priority: 20.07.2023 TW 112207566 U
(71) Applicant: Needleless Corporation, Taoyuan City 32658 (TW)
(72) Inventor: CHIEN, CHIH-WEI, Taoyuan City (TW)
(74) Representative: Durán-Corretjer, S.L.P.

(57) **Abstract**

A connector structure (Z) includes a first base element (1), a shielding element (2), and a sleeve element (3). The first base element (1) has a first accommodation space (10) and a first opening (11). A first inner wall (12) of the first base element (1) corresponding to the first accommodation space (10) protrudes outward to form a first pointed portion (13). The first pointed portion (13) has a first channel (130) and at least one first through hole (131). Another end of the first base element (1) protrudes outward to form a first protruding portion (14). The first protruding portion (14) has a second channel (140). The shielding element (2) is detachably sleeved on the first pointed portion (13) and located in the first accommodation space (10). The shielding element (2) has a second accommodation space (20), and one end of the shielding element (2) has an elastic gap (21). The sleeve element (3) is detachably connected to the first base element (1) and located in the first accommodation space (10).

## Description

### FIELD OF THE DISCLOSURE

The present invention relates to a connector structure, and more particularly to a connector structure having a simple structure and a small number of components.

### BACKGROUND OF THE DISCLOSURE

During injections, in order to prevent patients from repeatedly suffering the pain of needles piercing the skin, and facilitate medical staff to inject medicines to patients more easily and efficiently, medical connectors are generally mounted on the pipeline or on the upper cover of the measuring cylinder of drip devices. Therefore, the patient only needs to receive one injection, and medical staff can repeatedly add medicine to the patient through the connector.

Conventional medical connectors are mainly produced by assembling multiple small components. However, because these small components are too small in size, the number of processing times and processes required are more complicated and cumbersome, and the product yield and structural stability cannot be improved. Furthermore, the manufacturing cost is also relatively difficult to reduce.

### SUMMARY OF THE DISCLOSURE

In response to the above-referenced technical inadequacies, the present invention provides a connector structure.

In order to solve the above-mentioned problems, one of the technical aspects adopted by the present invention is to provide a connector structure. The connector structure includes a first base element, a shielding element, and a sleeve element. The first base element has a first accommodation space and a first opening. The first opening is located at one end of the first base element and corresponds to the first accommodation space. A first inner wall of the first base element corresponding to the first accommodation space protrudes outward to form a first pointed portion. The first pointed portion has a first channel and at least one first through hole. The at least one first through hole communicates with the first channel, a part of a body of the first pointed portion is exposed outside the first base element, and another end of the first base element protrudes outward to form a first protruding portion. The first protruding portion has a second channel, and the second channel communicates with the first channel. The shielding element is detachably sleeved on the first pointed portion and located in the first accommodation space. The shielding element has a second accommodation space, and the second accommodation space accommodates the first pointed portion. One end of the shielding element has an elastic gap, another end of the shielding element has a second opening, and the second opening communicates with the second accommodation space. The sleeve element is detachably connected to the first base element and located in the first accommodation space. The sleeve element has a third accommodation space, and the third accommodation space accommodates the shielding element.

In order to solve the above-mentioned problems, another one of the technical aspects adopted by the present invention is to provide a connector structure. The connector structure includes a second base element, a shielding element, and a sleeve element. One side of the second base element protrudes outward to form a plurality of first extension portions, and another side of the second base element protrudes outward to form an annular fixing portion. One of the first extension portions has a fifth accommodation space and a fifth opening, and the fifth opening is located at one end of the one of the first extension portions and corresponds to the fifth accommodation space. A fourth inner wall of the one of the first extension portions corresponding to the fifth accommodation space protrudes outward to form a second pointed portion, and the second pointed portion has a fourth channel and at least one second through hole. The at least one second through hole communicates with the fourth channel, the fourth channel penetrates a body of the second base element, and a part of a body of the second pointed portion is exposed outside the one of the first extension portions. The shielding element is detachably sleeved on the second pointed portion and located in the fifth accommodation space. The shielding element has a second accommodation space, and the second accommodation space accommodates the second pointed portion. One end of the shielding element has an elastic gap, another end of the shielding element has a second opening, and the second opening communicates with the second accommodation space. The sleeve element is detachably connected to the one of the first extension portions and located in the fifth accommodation space. The sleeve element has a third accommodation space, and the third accommodation space accommodates the shielding element.

Therefore, in the connector structure provided by the present invention, by virtue of the aforementioned technical solutions, product yield and structural stability of the connector structure can be improved, and a production cost of the connector structure can be lowered.

These and other aspects of the present invention will become apparent from the following description of the embodiment taken in conjunction with the following drawings and their captions, although variations and modifications therein may be affected without departing from the spirit and scope of the novel concepts of the disclosure.

### BRIEF DESCRIPTION OF THE DRAWINGS

The described embodiments may be better understood by reference to the following description and the accompanying drawings, in which:
FIG. 1 is a schematic perspective view of a connector structure according to a first embodiment of the present invention;
FIG. 2 is a schematic exploded view of the connector structure in one perspective according to the first embodiment of the present invention;
FIG. 3 is a schematic exploded view of the connector structure in another perspective according to the first embodiment of the present invention;
FIG. 4 is a schematic cross-sectional view taken along line IV-IV of FIG. 1;
FIG. 5 is a schematic view of the connector structure in use according to the first embodiment of the present invention;
FIG. 6 is a first schematic perspective view of the connector structure according to a second embodiment of the present invention;
FIG. 7 is a schematic exploded view of the connector structure according to the second embodiment of the present invention;
FIG. 8 is a schematic cross-sectional view taken along line VIII-VIII of FIG. 6;
FIG. 9 is a second schematic perspective view of the connector structure according to the second embodiment of the present invention;
FIG. 10 is a schematic cross-sectional view taken along line X-X of FIG. 9;
FIG. 11 is a schematic perspective view of the connector structure in one perspective according to a third embodiment of the present invention;
FIG. 12 is a schematic perspective view of the connector structure in another perspective according to the third embodiment of the present invention; and
FIG. 13 is a schematic cross-sectional view taken along line XIII-XIII of FIG. 11.

### DETAILED DESCRIPTION OF THE EXEMPLARY EMBODIMENTS

The present invention is more particularly described in the following examples that are intended as illustrative only since numerous modifications and variations therein will be apparent to those skilled in the art. Like numbers in the drawings indicate like components throughout the views. As used in the description herein and throughout the claims that follow, unless the context clearly dictates otherwise, the meaning of "a," "an" and "the" includes plural reference, and the meaning of "in" includes "in" and "on." Titles or subtitles can be used herein for the convenience of a reader, which shall have no influence on the scope of the present invention.

The terms used herein generally have their ordinary meanings in the art. In the case of conflict, the present document, including any definitions given herein, will prevail. The same thing can be expressed in more than one way. Alternative language and synonyms can be used for any term(s) discussed herein, and no special significance is to be placed upon whether a term is elaborated or discussed herein. A recital of one or more synonyms does not exclude the use of other synonyms. The use of examples anywhere in this specification including examples of any terms is illustrative only, and in no way limits the scope and meaning of the present invention or of any exemplified term. Likewise, the present invention is not limited to various embodiments given herein. Numbering terms such as "first," "second" or "third" can be used to describe various components, signals or the like, which are for distinguishing one component/signal from another one only, and are not intended to, nor should be construed to impose any substantive limitations on the components, signals or the like.

### [First Embodiment]

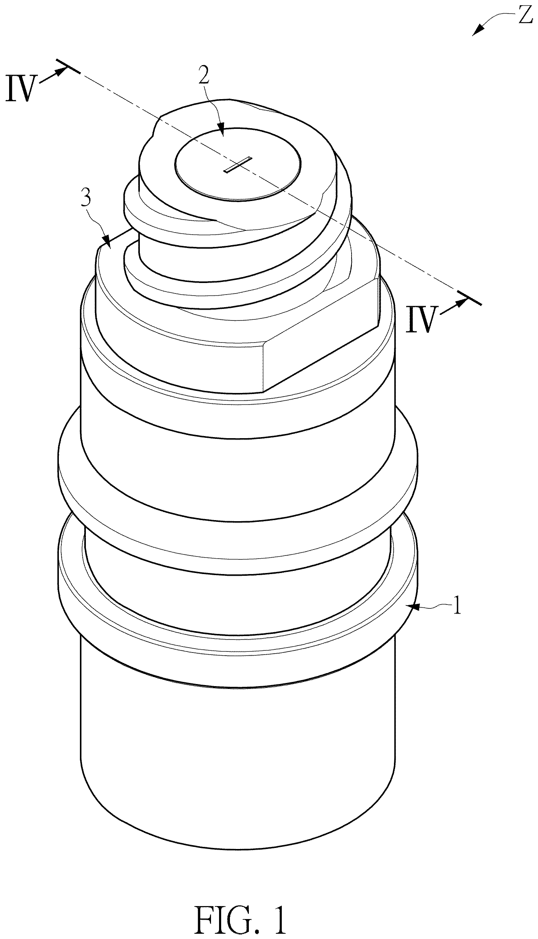

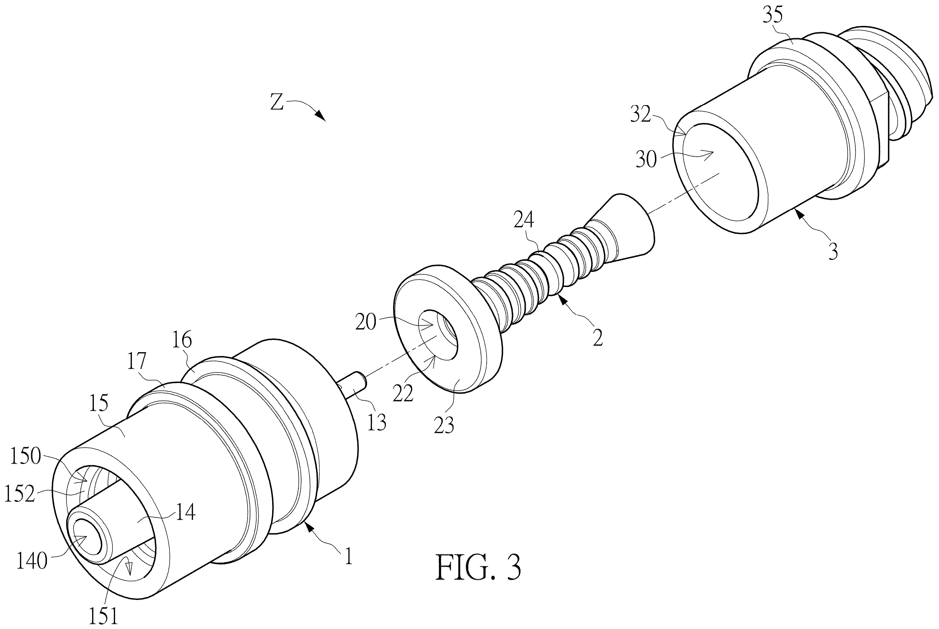

Referring to FIG. 1 to FIG. 5, which are respectively a schematic perspective view, a schematic exploded view in one perspective, a schematic exploded view in another perspective, a schematic cross-sectional view taken along line IV-IV of FIG. 1, and a schematic view of a connector structure in use, according to a first embodiment of the present invention. As shown in the above-mentioned figures, the first embodiment of the present invention provides a connector structure Z, and the connector structure Z includes a first base element 1, a shielding element 2, and a sleeve element 3.

As shown in FIG. 1 to FIG. 5, the first base element 1 can have a first accommodation space 10 and a first opening 11. The first opening 11 is located at one end of the first base element 1 and corresponds to the accommodation space 10. A first inner wall 12 of the first base element 1 that corresponds to the first accommodation space 10 protrudes outward to form a first pointed portion 13. The first pointed portion 13 can have a first channel 130 and at least one first through hole 131, the at least one first through hole 131 communicates with the first channel 130, a part of a body of the first pointed portion 13 is exposed outside the first base element 1, and another end of the first base element 1 protrudes outward to form a first protruding portion 14. The first protruding portion 14 can have a second channel 140, and the second channel 140 communicates with the first channel 130.

For example, the first base element 1 can be an integrated hollow structure, and the first base element 1 can have a first pointed portion 13 and the first protruding portion 14 that pass through the center of a body of the first base element 1. The cross-section of the first base element 1 can be trifurcated, but is not limited thereto. The first pointed portion 13 can have a tapered structure, but is not limited thereto. A front end of the first pointed portion 13 is exposed outside the first base element 1 and is not located in the first accommodation space 10. Furthermore, an outer edge of the another end of the first base element 1 can further extend outward to form an outer annular portion 15, and a fourth accommodation space 150 is defined in an interior of the outer annular portion 15. The first protruding portion 14 is located in the fourth accommodation space 150, and one end of the first protruding portion 14 is exposed outside the first base element 1 and is not located in the fourth accommodation space 150. A second inner wall 151 of the outer annular portion 15 corresponding to the fourth accommodation space 150 may also have at least one first connecting portion 152, and the first connecting portion 152 may be screw threads or other type of engaging and connecting structure.

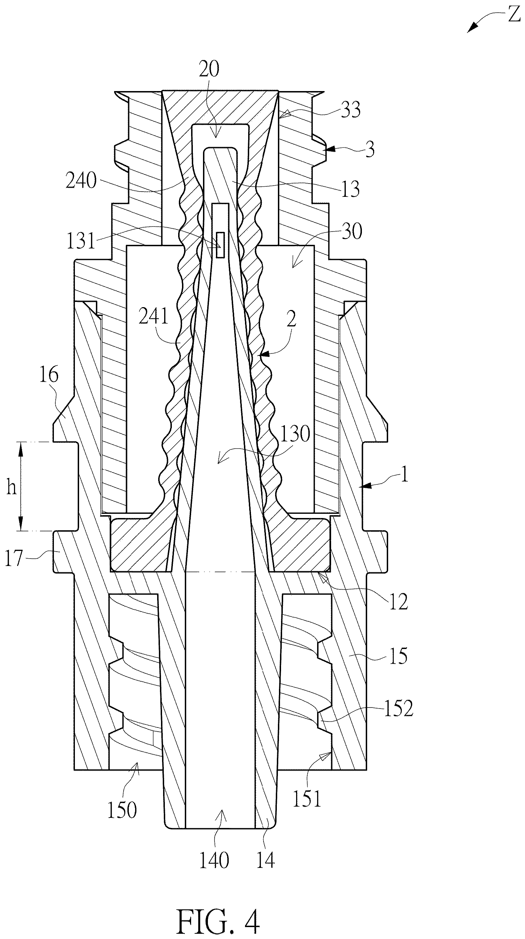

In addition, as shown in FIG. 4, the outer edge of the first base element 1 may further have a first annular portion 16 and a second annular portion 17. The first annular portion 16 and the second annular portion 17 are annularly provided on an outer edge of the first base element 1. The first annular portion 16 and the second annular portion 17 can have a predetermined distance h therebetween. The predetermined distance h can be between 2.8 mm and 3.4 mm, and preferably is 3.2 mm.

Then, as shown in FIG. 1 to FIG. 5, the shielding element 2 is detachably sleeved on the first pointed portion 13 and is located in the first accommodation space 10. The shielding element 2 has a second accommodation space 20, and the second accommodation space 20 can be used to accommodate the first pointed portion 13. One end of the shielding element 2 has an elastic gap 21, another end of the shielding element 2 has a second opening 22, and the second opening 22 communicates with the second accommodation space 20.

For example, the shielding element 2 can be made of an elastic material and have an integrated hollow structure. The shielding element 2 can have a base portion 23 and a tube body portion 24. One end of the tube body portion 24 is connected to the base portion 23. The tube body portion 24 may be hyperbola-shaped. Another end of the tube body portion 24 can have the elastic gap 21. When the elastic gap 21 is in an open state, the elastic gap 21 can communicate with the second accommodation space 20 and an external space of the tube body portion 24, and when the elastic gap 21 is in a closed state, the elastic gap 21 closes the second accommodation space 20. The tube body portion 24 and an interior of the base portion 23 may jointly form the second accommodation space 20, and one side of the base portion 23 facing away from the tube body portion can have the second opening 22. The shielding element 2 can be sleeved on the first pointed portion 13 through the second opening 22 to cover the first pointed portion 13. Furthermore, the tube body portion 24 can be divided into a front end portion 240 and a main body 241. The front end portion 240 can be in a trumpet shape or a cone shape, the main body 241 can be in a cone shape, and an outer surface and an inner wall surface of the main body 241 are wavy (that is, have concave and convex structures).

Accordingly, as shown in FIG. 1 to FIG. 5, the sleeve element 3 is detachably connected to the first base element 1 and is located in the first accommodation space 10. The sleeve element 3 can have a third accommodation space 30, and the third accommodation space 30 can be used to accommodate the shielding element 2.

For example, one end of the sleeve element 3 can have a third opening 31, and another end of the sleeve element 3 can have a fourth opening 32. The third opening 31 and the fourth opening 32 communicate with the third accommodation space 30. Moreover, an outer edge of one end of the sleeve element 3 can have at least one second connecting portion 34 (such as screw threads or other types of engagement and connection structures ), and the outer edge of the first base element 1 can have an annular stopper 35.

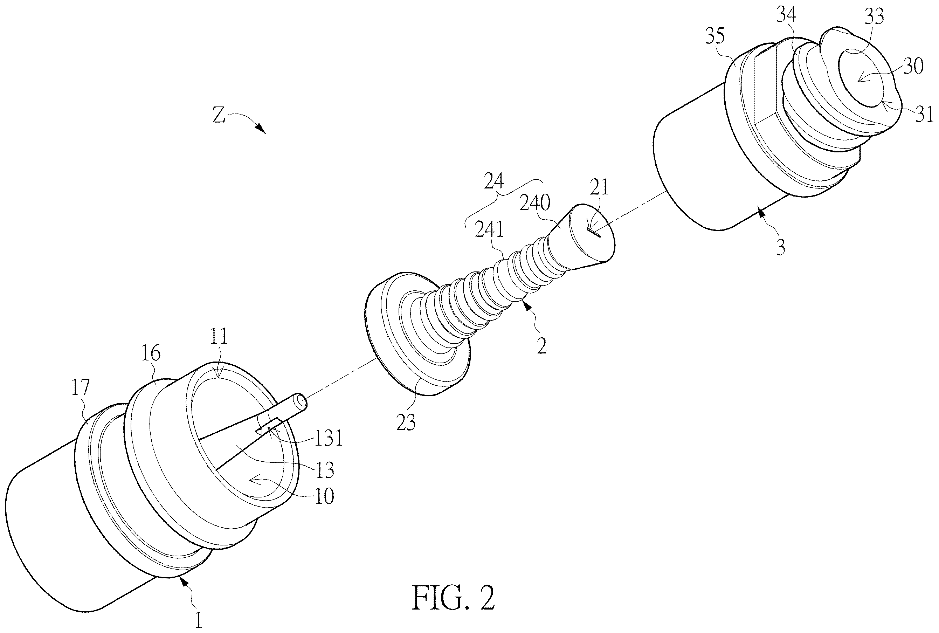

Therefore, as shown in FIG. 2 to FIG. 4, when the connector structure Z of the present invention is assembled, the shielding element 2 can be sleeved on the first pointed portion 13 of the first base element 1. At this time, the inner wall surface of the tube body portion 24 of the shielding element 2 contacts the first pointed portion 13, such that the first pointed portion 13 and the second accommodation space 20 form a closed space. It is worth noting that, the size of the second accommodation space 20 formed by the inner wall surface of the tube body portion 24 (that is, the size of the space surrounded by the inner wall surface of the tube body portion 24) may be smaller than an outer edge size of the first pointed portion 13. Therefore, when the shielding element 2 is sleeved on the first pointed portion 13, the inner wall surface of the tube body portion 24 can closely contact and abut against the outer edge (i.e., an outer surface) of the first pointed portion 13.

Then, the sleeve element 3 is assembled with the first base element 1. At this time, the sleeve element 3 will be sleeved on the shielding element 2 to accommodate the shielding element 2 and the first pointed portion 13 in the third accommodation space 30. An outer edge of the one end of the shielding element 2 will be attached to a third inner wall 33 of the sleeve element 3, such that the one end of the shielding element 2 and the one end of the sleeve element 3 do not have a gap therebetween. That is, the third accommodation space 30 is closed. The surface of the one end of the shielding element 2 will be coplanar with the surface of the one end of the sleeve element 3 (that is, appearing to be on the same plane). Moreover, when the sleeve element 3 is connected to the first base element 1, the first base element 1 contacts and abuts against the annular stopper 35.

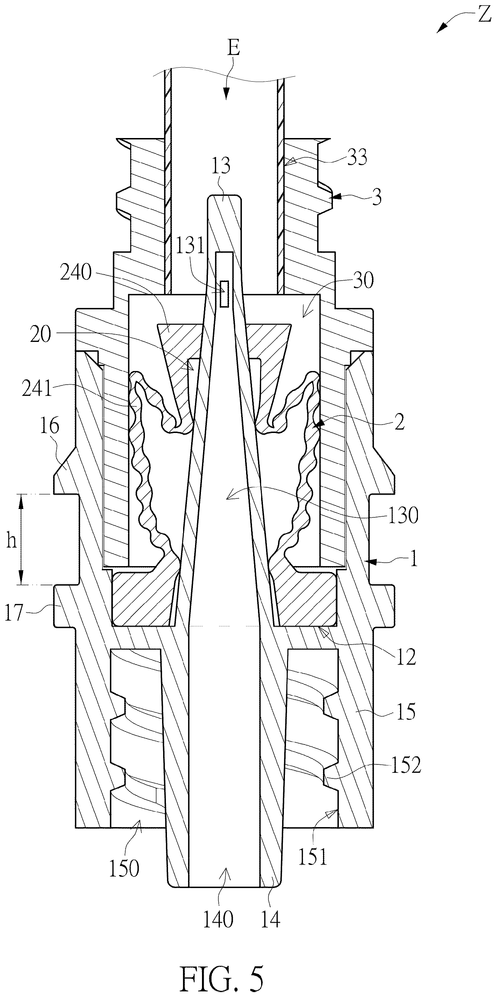

Furthermore, as shown in FIG. 1 to FIG. 5, when a user uses the connector structure Z of the present invention, the connector structure Z can be connected to multiple external devices; that is, the user can insert one of external devices E (such as a fluid supply device, but is not limited thereto) into an interior of the connector structure Z through a front end of the connector structure Z, and connect a rear end of the connector structure Z with another external device (such as an injection device, but is not limited thereto; not shown in the figure). At this time, a front end of the one of the external devices E will penetrate deeply into the third accommodation space 30 through the third opening 31 of the sleeve element 3, and at the same time squeeze and push the shielding element 2. Then, since the another end of the tube body portion 24 will be squeezed and pushed by the one of the external devices E, the tube body portion 24 shrinks and deforms. That is, the tube body portion 24 is changed from an initial state (as shown in FIG. 4) to a deformed state (as shown in FIG. 5). Furthermore, the another end of the tube body portion 24 will be displaced toward the base portion 23, such that the front end of the first pointed portion 13 passes through the elastic gap 21 and is exposed outside the shielding element 2. At this time, the first through hole 131 of the first pointed portion 13 is also exposed outside the shielding element 2. That is to say, when the one end of the shielding element 2 is pushed by an external force and displaces toward the another end of the shielding element 2, the one end of the shielding element 2 drives a part of the body of the first pointed portion 13 and the at least one first through hole 131 to pass through the elastic gap 21 and be exposed outside the shielding element 2.

Next, the one of the external devices E will be connected to the first pointed portion 13. At this time, the one of the external devices E can supply fluid (such as medical solution, but is not limited thereto; not shown in the figure) to the first pointed portion 13, thereby allowing the fluid to flow into the first channel 130 through the first through hole 131 of the first pointed portion 13. Since another one of the external devices is connected to the first protruding portion 14, the fluid can flow into another external device through the first channel 130 and the second channel 140. Another external device can be connected to the first protruding portion 14 through the first connecting portion 152 being connected to the outer annular portion 15.

Correspondingly, when the user wants to replace the external device or stops using the connector structure Z, the user can pull out or yank out one of the external devices E from the connector structure Z, or detach the another external device E from the first protruding portion 14. When the user pulls out or yanks out one of the external devices E, the tube body portion 24 of the shielding element 2 can use an elastic restoring force to cause the other end of the tube body portion 24 to displace toward the third opening 31 of the sleeve element 3. When the other end of the tube body portion 24 is displaced, the front end of the first pointed portion 13 will pass through the elastic gap 21 again and return to the second accommodation space 20. That is, when the external force pushing against the shielding element 2 is eliminated, the shielding element 2 drives one end of the shielding element 2 to displace in a direction away from the other end of the shielding element 2 through the elastic restoring force, so as to drive the part of the body of the first pointed portion 13 and the at least one first through hole 131 to pass through the elastic gap 21 again to be located in the second accommodation space 20. Then, the tube body portion 24 will transform from the deformed state back to the initial state, and the outer edge of the other end of the tube body portion 24 will be attached to the third inner wall 33 of the sleeve element 3.

In this way, the connector structure Z of the present invention uses the above-mentioned technical solutions and provides a sleeve element 3 that is a single-component to simplify the number of components of the connector structure Z; therefore, during the production of the connector structure Z, product yield and structural stability of the connector structure Z can be improved, and a production cost of the connector structure Z can be lowered.

### [Second Embodiment]

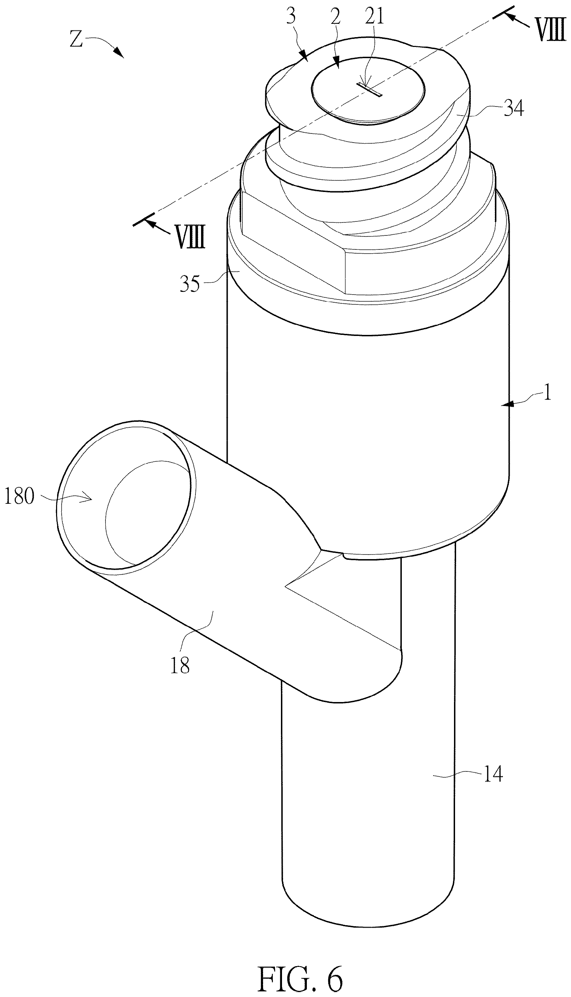

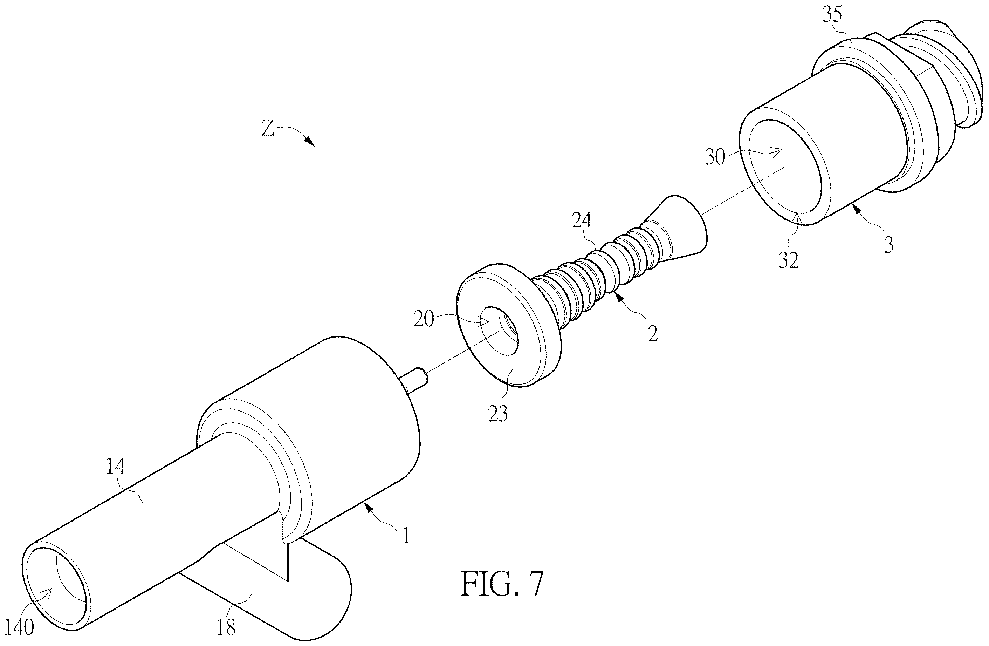

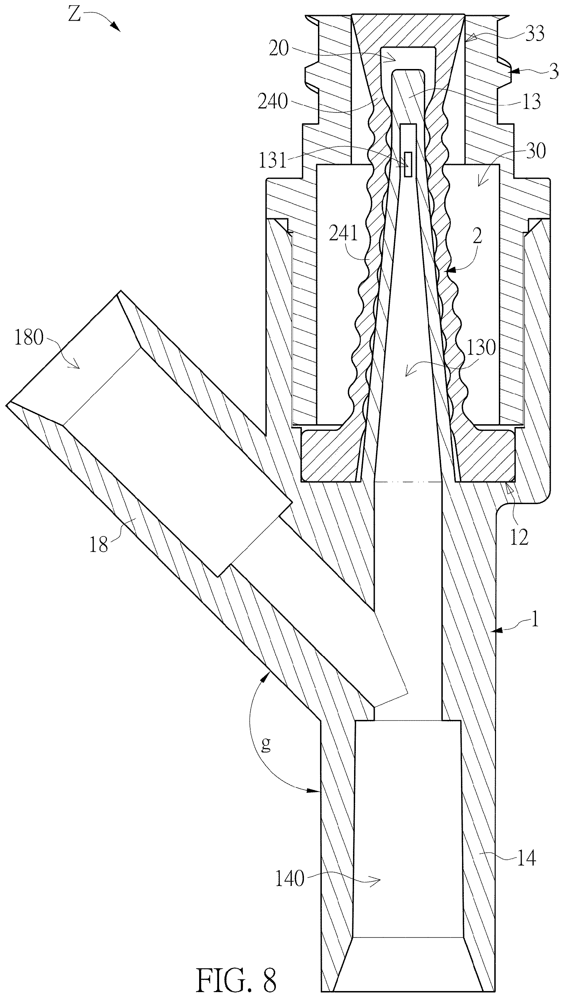

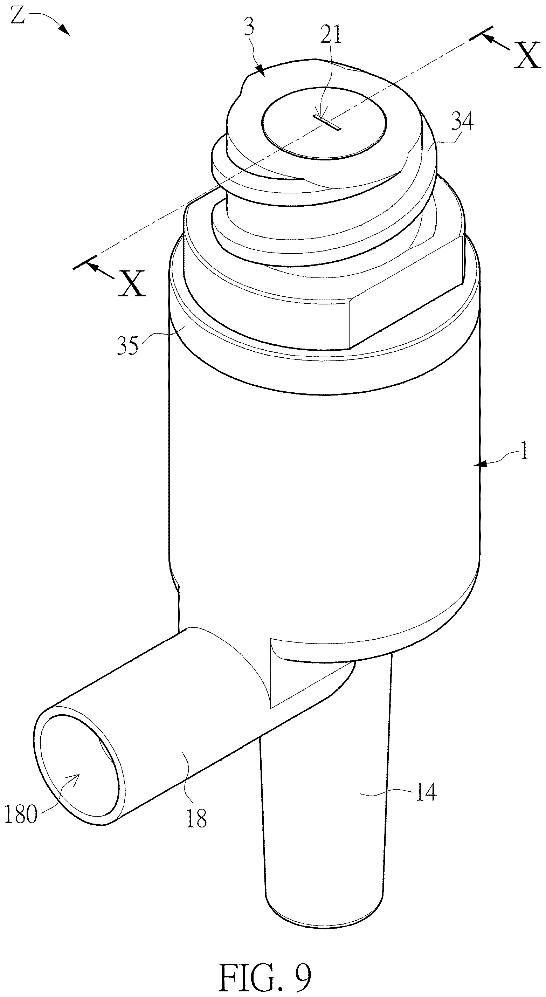

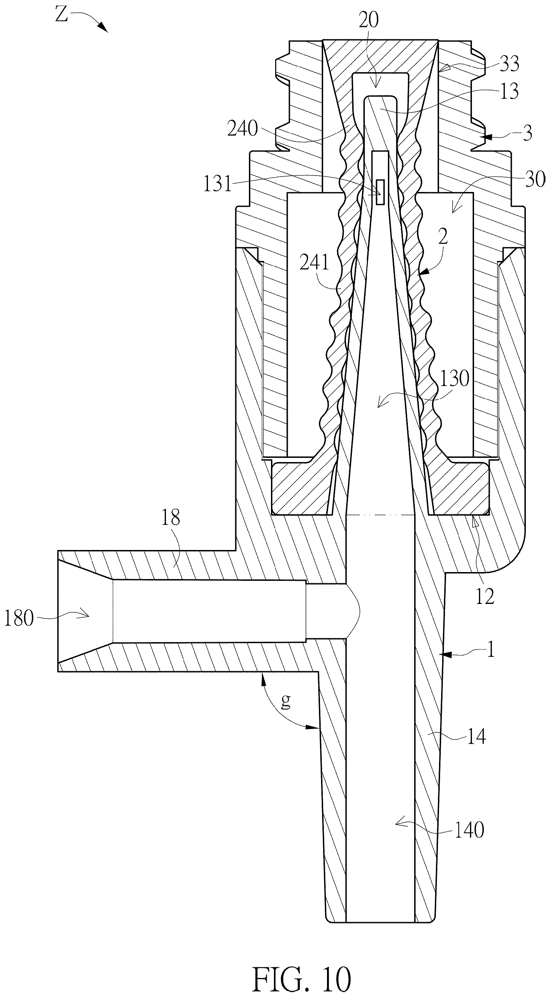

Referring to FIG. 6 to FIG. 10, which are respectively a first schematic perspective view, a schematic exploded schematic view, a schematic cross-sectional view taken along line VIII-VIII of FIG. 6, a second schematic perspective view of the connector structure of a second embodiment of the present invention, and a schematic cross-sectional view taken along line X-X of FIG. 9, and reference can be further made in conjunction to FIG. 1 to FIG. 5. As shown in the figure, the connector structure Z of this embodiment is substantially similar to the connector structure Z of the above-mentioned embodiment. Therefore, the arrangement or operation of the same elements will not be reiterated herein. The difference between this embodiment and the above-mentioned first embodiment is that, in this embodiment, an outer edge of the first protruding portion 14 of the first base element 1 can protrude outward to form a second protruding portion 18. The second protruding portion 18 can have a third channel 180, and the third channel 180 communicates with the second channel 140. The first protruding portion 14 and the second protruding portion 18 may define a predetermined included angle g therebetween, and the predetermined included angle g may range from 90 degrees to 150 degrees. Preferably, the predetermined included angle g is 90 degrees or 135 degrees.

For example, as shown in FIG. 6 to FIG. 10, the second protruding portion 18 can protrude outward from the bottom of the connector structure Z of the present invention for being connected to other external devices (such as a fluid supply device, an injection device, or fluid transmission pipes, but is not limited thereto; not shown in the figure). The third channel 180 of the second protruding portion 18 communicates with the second channel 140 of the first protruding portion 14. Therefore, after one of the external devices E supplies the fluid into the first channel 130 of the first pointed portion 13, the fluid can be transmitted to another external device and other external devices via the second channel 140 and the third channel 180.

### [Third Embodiment]

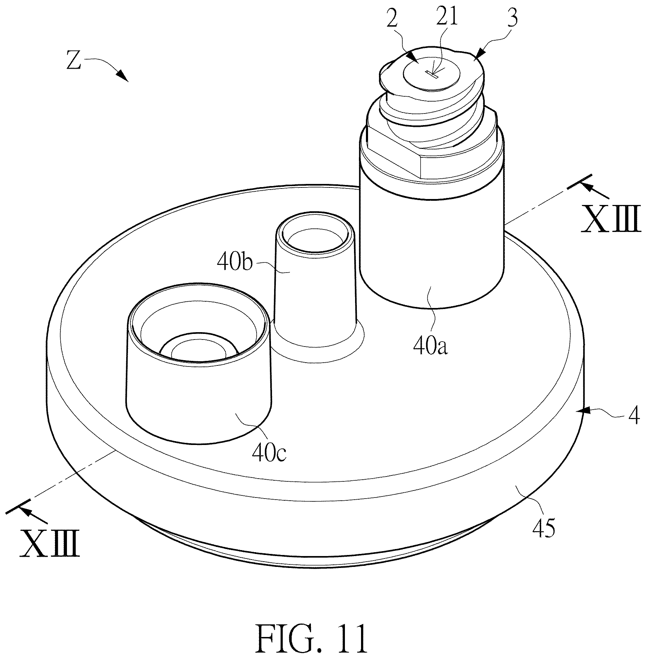

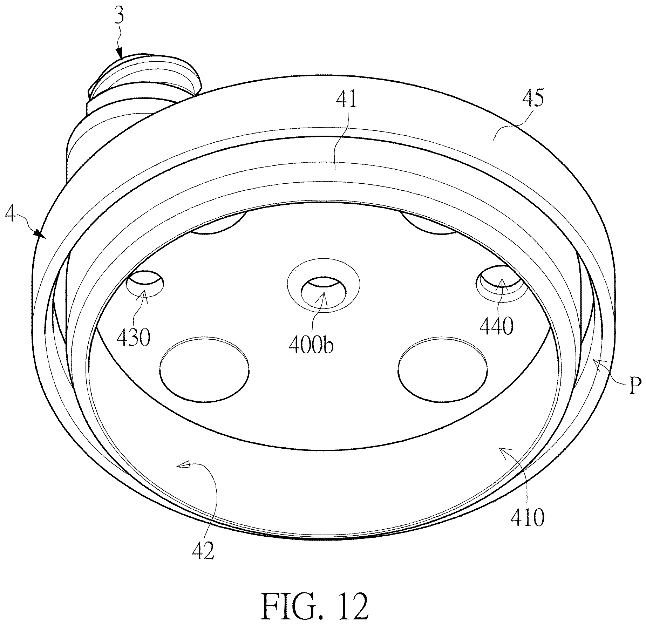

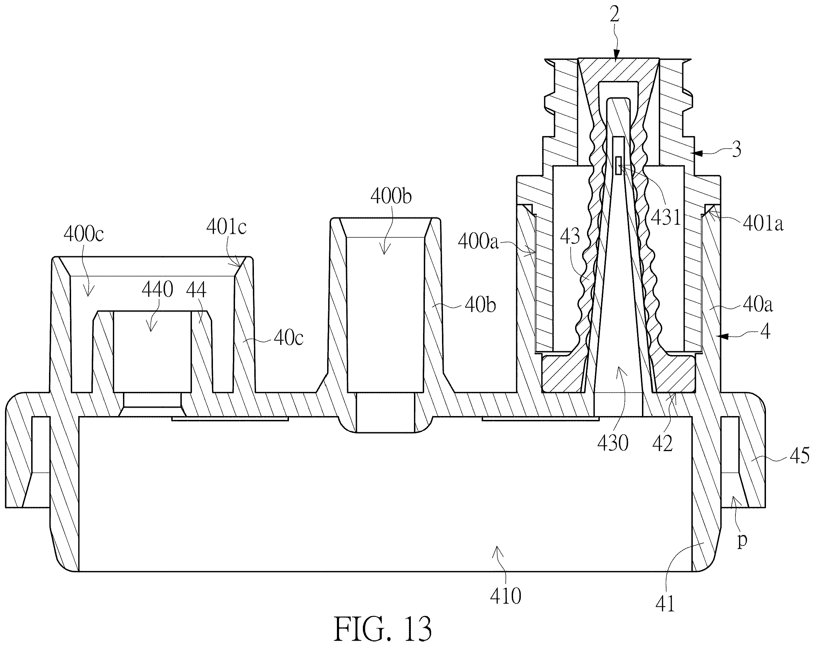

Referring to FIG. 11 to FIG. 13, which are respectively a schematic perspective view, a schematic perspective view of the connector structure in another perspective according to a third embodiment of the present invention, and a schematic cross-sectional view taken along line XIII-XIII of FIG. 11, and reference can be further made in conjunction to FIG. 1 to FIG. 10. As shown in the abovementioned figures, the invention further provides a connector structure Z that include a second base element 4, a shielding element 2, and a sleeve element 3.

As shown in FIG. 1 to FIG. 5, and FIG. 11 to FIG. 13, one side of the second base element 4 protrudes outward to form a plurality of first extension portions 40a, 40b, and 40c, and another side of the second base element 4 protrudes outward to form an annular fixing portion 41. One of the first extension portions 40a is recessed inward to define a fifth accommodation space 400a and a fifth opening 401a. The fifth opening 401a is located at one end of one of the first extension portions 40a and corresponds to the fifth accommodation space 400a. A fourth inner wall 42 of the second base element 4 corresponding to the fifth accommodation space 400a protrudes outward to form a second pointed portion 43. The second pointed portion 43 can have a fourth channel 430 and at least one second through hole 431. The at least one second through hole 431 communicates with the fourth channel 430. The fourth channel 430 penetrates a body of the second base element 4, and a part of a body of the second pointed portion 43 is exposed outside the one of the first extension portions 40a.

For example, one side of the second base element 4 of the present invention can protrude outward to form the plurality of first extension portions 40a, 40b, and 40c. In this embodiment, the three first extension portions 40a, 40b, and 40c are arranged in a straight line as an example, but are not limited thereto. In practical applications, two, three, or more first extension portions can also be used, and arranged in an array, a geometric shape, or an irregular arrangement. The fifth accommodation space 400a, the fifth opening 401a, the fourth inner wall 42, the second pointed portion 43, the fourth channel 430, and the second through hole 431 of the second base element 4 are substantially the same as the first accommodation space 10, the first opening 11, the first inner wall 12, the first pointed portion 13, the first channel 130, and the first through hole 131 of the first base element 1 of the above-mentioned embodiments. Therefore, the arrangement or operation of the same elements will not be reiterated herein. The first extension portion 40b may be located at the center of the second base element 4, and the first extension portion 40b can have a fifth channel 400b penetrating the body of the second base element 4. The first extension portion 40c corresponds to the first extension portion 40a and is away from the first extension portion 40a, and the first extension portion 40b is located between the first extension portion 40c and the first extension portion 40a. The first extension portion 40c is recessed inward to form a sixth accommodation space 400c and a sixth opening 401c. The fourth inner wall 42 of the second base element 4 corresponding to the sixth accommodation space 400c protrudes outward to form a second extension portion 44. The second extension portion 44 can have a sixth channel 440 penetrating the body of the second base element 4. The specific implementation of the first extension portion 40a is substantially the same as in the specific implementation of the first base element 1 of the first embodiment, and therefore will not be described in detail herein. The first extension portion 40b and the first extension portion 40c can respectively be detachably connected to an external device (such as fluid supply devices, but are not limited thereto; not shown in the figure).

Moreover, the second base element 4 can also be detachably connected to other external devices (such as fluid supply devices, but are not limited thereto; not shown in the figure) through the annular fixing portion 41. Furthermore, a side edge of the body of the second base element 4 can also be bent and extended outward to form a bending limiting portion 45. The cross-section of the bending limiting portion 45 can be arc-shaped, L-shaped, or in other curved shapes, and the bending limiting portion 45 and the annular fixing portion 41 have a snap gap p therebetween. Therefore, the second base element 4 can be clamped to external devices through the bending limiting portion 45 and the annular fixing portion 41. That is, a part of a body of the external device enters the snap gap p to be firmly assembled with the external device.

Next, as shown in FIG. 1 to FIG. 5, and FIG. 11 to FIG. 13, the shielding element 2 is detachably sleeved on the second pointed portion 43 and located in the fifth accommodation space 400a. The shielding element 2 can have a second accommodation space 20. The second accommodation space 20 is used to accommodate the second pointed portion 43. One end of the shielding element 2 can have an elastic gap 21, and another end of the shielding element 2 can have a second opening 22. The second opening 22 communicates with the second accommodation space 20. The sleeve element 3 is detachably connected to the one of the first extension portions 40a and is located in the fifth accommodation space 400a. The sleeve element 3 can have a third accommodation space 30, and the third accommodation space 30 is used to accommodate the shielding element. 2. In this embodiment, the shielding element 2 and the sleeve element 3 are substantially the same as the shielding element 2 and the sleeve element 3 of the above embodiments. Therefore, the arrangement or operation of the same elements will not be reiterated herein.

Furthermore, the second base element 4 has an integrated structure. When one end of the shielding element 2 is pushed by an external force and displaces toward the another end of the shielding element 2, the one end of the shielding element 2 drives a part of the body of the second pointed portion 43 and the at least one second through hole 431 to pass through the elastic gap 21 and be exposed outside the shielding element 2. For specific implementations, reference can be made to the foregoing implementations, and details will not be reiterated herein.

Moreover, when the external force pushing against the shielding element 2 is eliminated, the shielding element 2 drives the one end of the shielding element 2 to displace in a direction away from the another end through an elastic restoring force, so as to drive the part of the body of the second pointed portion 43 and the at least one second through hole 431 to pass through the elastic gap 21 again and be located in the second accommodation space 20. For specific implementations, reference can be made to the foregoing implementations, and details will not be reiterated herein.

In addition, the shielding element 2 can have a base portion 23 and a tube body portion 24. One end of the tube body portion 24 is connected to the base portion 23. The tube body portion 24 is hyperbola-shaped. Another end of the tube body portion 24 can have an elastic gap 21, the tube body portion 24 and an interior of the base portion 23 jointly form the second accommodation space 20, and one side of the base portion 23 facing away from the tube body portion 24 can have the second opening 22. For specific implementations, reference can be made to the foregoing implementations, and details will not be reiterated herein.

However, the above example is only one of the possible embodiments and is not intended to limit the present invention.

### [Beneficial Effects of the Embodiments]

One of the beneficial effects of this invention is that, in the connector structure provided by the present invention, by virtue of the aforementioned technical solutions, product yield and structural stability of the connector structure can be improved, and a production cost of the connector structure can be lowered.

Furthermore, the connector structure Z of the present invention uses the above-mentioned technical solution to simplify the number of components of the connector structure Z by providing a single-component sleeve element 3, such that, during the production of the connector structure Z, product yield and structural stability of the connector structure Z can be improved, and a production cost of the connector structure Z can be lowered. Moreover, by providing the second protruding portion 18, the number of external devices that can be connected to the connector structure Z of the present invention can also be increased.

The foregoing description of the exemplary embodiments of the disclosure has been presented only for the purposes of illustration and description and is not intended to be exhaustive or to limit the disclosure to the precise forms disclosed. Many modifications and variations are possible in light of the above teaching.

The embodiments were chosen and described in order to explain the principles of the disclosure and their practical application so as to enable others skilled in the art to utilize the disclosure and various embodiments and with various modifications as are suited to the particular use contemplated. Alternative embodiments will become apparent to those skilled in the art to which the present invention pertains without departing from its spirit and scope.

## Claims

1. A connector structure (Z), **characterized by** comprising:
a first base element (1) having a first accommodation space (10) and a first opening (11), wherein the first opening (11) is located at one end of the first base element (1) and corresponds to the first accommodation space (10); wherein a first inner wall (12) of the first base element (1) corresponding to the first accommodation space (10) protrudes outward to form a first pointed portion (13); wherein the first pointed portion (13) has a first channel (130) and at least one first through hole (131); wherein the at least one first through hole (131) communicates with the first channel (130), a part of a body of the first pointed portion (13) is exposed outside the first base element (1), and another end of the first base element (1) protrudes outward to form a first protruding portion (14); wherein the first protruding portion (14) has a second channel (140), and the second channel (140) communicates with the first channel (130);
a shielding element (2) detachably sleeved on the first pointed portion (13) and located in the first accommodation space (10), wherein the shielding element (2) has a second accommodation space (20), and the second accommodation space (20) accommodates the first pointed portion (13); wherein one end of the shielding element (2) has an elastic gap (21), another end of the shielding element (2) has a second opening (22), and the second opening (22) communicates with the second accommodation space (20); and
a sleeve element (3) detachably connected to the first base element (1) and located in the first accommodation space (10), wherein the sleeve element (3) has a third accommodation space (30), and the third accommodation space (30) accommodates the shielding element (2).

2. The connector structure according to claim 1, wherein an outer edge of the another end of the first base element (1) extends outward to form an outer annular portion (15), and a fourth accommodation space (150) is defined in an interior of the outer annular portion (15); wherein the first protruding portion (14) is located in the fourth accommodation space (150), and a second inner wall (151) of the outer annular portion (15) corresponding to of the fourth accommodation space (150) has at least one first connecting portion (152).

3. The connector structure according to claim 1, wherein the first base element (1) is an integrated structure; wherein an outer edge of the first base element (1) has a first annular portion (16) and a second annular portion (17), and the first annular portion (16) and the second annular portion (17) have a predetermined distance (h) therebetween.

4. The connector structure according to claim 1, wherein an outer edge of the first protruding portion (14) of the first base element (1) protrudes outward to form a second protruding portion (18), the second protruding portion (18) has a third channel (180), and the third channel (180) communicates with the second channel (140); wherein the first protruding portion (14) and the second protruding portion (18) define a predetermined included angle (g) therebetween, and the predetermined included angle (g) ranges from 90 degrees to 150 degrees.

5. The connector structure according to claim 4, wherein the predetermined included angle (g) is 90 degrees or 135 degrees.

6. The connector structure according to claim 1, wherein, when the one end of the shielding element (2) is pushed by an external force and displaces toward the another end of the shielding element (2), the one end of the shielding element (2) drives the part of the body of the first pointed portion (13) and the at least one first through hole (131) to pass through the elastic gap (21) and be exposed outside the shielding element (2).

7. The connector structure according to claim 6, wherein, when the external force that pushes the shielding element (2) is eliminated, the shielding element (2) drives the one end of the shielding element (2) to displace in a direction away from the another end of the shielding element (2) through an elastic restoring force, so as to drive the part of the body of the first pointed portion (13) and the at least one first through hole (131) to pass through the elastic gap (21) again and be located in the second accommodation space (20).

8. The connector structure according to claim 1, wherein the shielding element (2) has a base portion (23) and a tube body portion (24), one end of the tube body portion (24) is connected to the base portion (23), and the tube body portion (24) is hyperbola-shaped; wherein another end of the tube body portion (24) has the elastic gap (21), the tube body portion (24) and an interior of the base portion (23) jointly form the second accommodation space (20), and one side of the base portion (23) facing away from the tube body portion (24) has the second opening (22).

9. The connector structure according to claim 1, wherein one end of the sleeve element (3) has a third opening (31), another end of the sleeve element (3) has a fourth opening (32), the third opening (31) and the fourth opening (32) communicate with the third accommodation space (30); wherein an outer edge of the one end of the shielding element (3) is attached to a third inner wall (33) of the sleeve element (3).

10. The connector structure according to claim 1, wherein an outer edge of one end of the sleeve element (3) has at least one second connecting portion (34), and the outer edge of the first base element (1) has an annular stopper (35); wherein, when the sleeve element (3) is connected to the first base element (1), the first base element (1) contacts the annular stopper (35).

11. A connector structure (Z), **characterized by** comprising:
a second base element (4), wherein one side of the second base element (4) protrudes outward to form a plurality of first extension portions (40a, 40b, 40c), and another side of the second base element (4) protrudes outward to form an annular fixing portion (41); wherein one of the first extension portions (40a) has a fifth accommodation space (400a) and a fifth opening (401a), and the fifth opening (401a) is located at one end of the one of the first extension portions (40a) and corresponds to the fifth accommodation space (400a); wherein a fourth inner wall (42) of the one of the first extension portions (40a) corresponding to the fifth accommodation space (400a) protrudes outward to form a second pointed portion (43), and the second pointed portion (43) has a fourth channel (430) and at least one second through hole (431); wherein the at least one second through hole (431) communicates with the fourth channel (430), the fourth channel (430) penetrates a body of the second base element (4), and a part of a body of the second pointed portion (43) is exposed outside the one of the first extension portions (40a);
a shielding element (2) detachably sleeved on the second pointed portion (43) and located in the fifth accommodation space (400a), wherein the shielding element (2) has a second accommodation space (20), and the second accommodation space (20) accommodates the second pointed portion (43); wherein one end of the shielding element (2) has an elastic gap (21), another end of the shielding element (2) has a second opening (22), and the second opening (22) communicates with the second accommodation space (20); and
a sleeve element (3) detachably connected to the one of the first extension portions (40a) and located in the fifth accommodation space (400a), wherein the sleeve element (3) has a third accommodation space (30), and the third accommodation space (30) accommodates the shielding element (2).

12. The connector structure according to claim 11, wherein the second base element (4) is an integrated structure; wherein, when one end of the shielding element (2) is pushed by an external force and displaces toward the another end of the shielding element (2), the one end of the shielding element (2) drives a part of the body of the second pointed portion (43) and the at least one second through hole (431) to pass through the elastic gap (21) and be exposed outside the shielding element (2).

13. The connector structure according to claim 12, wherein when the external force that pushes against the shielding element (2) is eliminated, the shielding element (2) drives the one end of the shielding element (2) to displace in a direction away from the another end of the shielding element (2) through an elastic restoring force, so as to drive the part of the body of the second pointed portion (43) and the at least one second through hole (431) to pass through the elastic gap (21) again and be located in the second accommodation space (20).

14. The connector structure according to claim 11, wherein the shielding element (2) has a base portion (23) and a tube body portion (24), one end of the tube body portion (24) is connected to the base portion (23), and the tube body portion (24) is hyperbola-shaped; wherein another end of the tube body portion (24) has the elastic gap (21), the tube body portion (24) and an interior of the base portion (23) jointly form the second accommodation space (20), and one side of the base portion (23) facing away from the tube body portion (24) has the second opening (22).
